# EUROPÄISCHE PATENTSCHRIFT

(11) **EP 4 663 152 B1**
(45) Veröffentlichungstag und Bekanntmachung des Hinweises auf die Patenterteilung: **26.08.2026**
(21) Anmeldenummer: 24181969.7
(22) Anmeldetag: 13.06.2024
(51) Int. Cl.: A61B 90/70, B08B 3/04

(54) **AUFBEREITUNGSEINHEIT FÜR MEDIZINPRODUKTE**
PROCESSING UNIT FOR MEDICAL DEVICES
UNITÉ DE CONDITIONNEMENT POUR DISPOSITIFS MÉDICAUX

(43) Veröffentlichungstag der Anmeldung: 17.12.2025
(73) Patentinhaber: Chemische Fabrik Dr. Weigert GmbH & Co. KG, 20539 Hamburg (DE)
(72) Erfinder: PREUGSCHAS, Michael, 22117 Hamburg (DE); HOCH, Detlev, 21720 Grünendeich (DE)
(74) Vertreter: GLAWE DELFS MOLL

(56) Entgegenhaltungen:
- EP-A1- 2 168 606
- EP-A1- 3 348 283
- DE-A1- 102010 012 872
- ARBEITSKREIS BETTGESTELL-UND WAGEN- DEKONTAMINATIONSANLAGEN: "Maschinelle Dekontamination", 1 January 2009 (2009-01-01), pages 20 - 1, XP055389743, Retrieved from the Internet <URL:http://www.meiko.de/fileadmin/Produkte/BA/AK-BWA_Broschuere_Maschinelle_Dekontamination.pdf> [retrieved on 20170711]

## Beschreibung

Die Erfindung betrifft eine Aufbereitungseinheit für Medizinprodukte, mit wenigstens zwei Aufbereitungsvorrichtungen für Medizinprodukte, wobei jeder Aufbereitungsvorrichtung eine Dosiervorrichtung mit wenigstens einem Vorlagebehälter und wenigstens einer Dosierpumpe zur Dosierung flüssiger Komponenten in den wenigstens einen Vorlagebehälter der Aufbereitungsvorrichtung zugeordnet ist, und wobei die Aufbereitungsvorrichtungen aus Vorratsbehältern gespeist werden.

Chirurgische Instrumente sowie andere medizinische Geräte werden im Krankenhaus oder in anderen medizinischen Einrichtungen üblicherweise maschinell in eine Aufbereitungsvorrichtung, insbesondere einem Reinigung- und Desinfektionsgerät (RDG), gereinigt und anschließend chemisch oder thermisch desinfiziert. Diese Wiederaufarbeitung medizinischer und chirurgischer Instrumente geschieht häufig in einer sogenannten Aufbereitungseinheit für Medizinprodukte (AEMP). Dort werden, gegebenenfalls nach einer lokalen Vorreinigung, Instrumente gereinigt, desinfiziert und gegebenenfalls sterilisiert.

EP 3 348 283 A1 beschreibt eine Aufbereitungseinheit für Medizinprodukte mit einer Aufbereitunsvorrichtung. Mehrere gegebenenfalls räumlich verteilte Reinigungs- und Desinfektionsgeräte (Aufbereitungsvorrichtungen) können von einer zentralen Dosierstation aus mit Reinigungs- und Desinfektionsmitteln versorgt werden. Eine solche zentrale Dosierstation erleichtert die Bereitstellung der nötigen Reinigungs- und Desinfektionsmittel, die kontinuierliche Überwachung der ausreichenden Lagerhaltung sowie die Dokumentation durchgeführter Reinigungsvorgänge.

Der Erfindung liegt die Aufgabe zugrunde, eine Aufbereitungseinheit der eingangs genannten Art zu schaffen, die eine flexible, kostengünstige und umweltfreundliche Aufarbeitung medizinischer Instrumente und Apparate ermöglicht.

Gelöst wird diese Aufgabe bei einer eingangs genannten Aufbereitungseinheit dadurch, dass zwischen Vorratsbehälter und den wenigstens zwei Aufbereitungsvorrichtungen wenigstens ein Zwischenbehälter vorgesehen ist, der mit wenigstens einer flüssigen Komponente aus wenigstens einem Vorratsbehälter gespeist wird und aus dem die Vorlagebehälter der Aufbereitungsvorrichtungen gespeist werden, wobei der wenigstens eine Zwischenbehälter drucklos ausgebildet ist, so dass er eine hydraulische Trennung des wenigstens einen Vorratsbehälters von den wenigstens zwei Aufbereitungsvorrichtungen bewirkt.

Zunächst seien einige im Rahmen der Erfindung verwendete Begriffe erläutert.

Eine Aufbereitungseinheit für Medizinprodukte ist die Gesamtanordnung umfassend wenigstens zwei Aufbereitungsvorrichtungen, insbesondere Reinigung- und Desinfektionsgeräten (RDG) sowie entsprechende Vorratsbehälter für flüssige Reinigung- und/oder Desinfektionskomponenten bzw. -mittel. Die Aufbereitungseinheit muss erfindungsgemäß nicht räumlich an einem Ort zusammengefasst sein, es können bspw. mehrere an unterschiedlichen Orten befindliche Aufbereitungsvorrichtungen von einer sogenannten Zentraldosierstation mit Reinigungs/Desinfektionsmittel gespeist werden. Entscheidend für die Bezeichnung als Aufbereitungseinheit ist der funktionale Zusammenhang der anspruchsgemäßen Bestandteile.

Eine Aufbereitungsvorrichtung für Medizinprodukte bezeichnet erfindungsgemäß eine Vorrichtung, mit der benutzte Medizinprodukte, insbesondere medizinische Instrumente und Apparate, teilweise oder vollständig in einen erneut gebrauchsfertigen Zustand versetzt werden. Teilweise bedeutet im Rahmen der Erfindung, dass wenigstens eine Reinigung und/oder Desinfektion durchgeführt wird, vollständig bedeutet im Rahmen der Erfindung eine zusätzliche Sterilisation, so dass ein steriler Zustand hergestellt wird. Bei einer solchen Aufbereitungsvorrichtung kann es sich insbesondere um Reinigungs- und Desinfektionsgeräte für medizinische Instrumente und Apparate gemäß ISO 15883 handeln.

Die Vorratsbehälter enthalten flüssige Reinigungs- und/oder Desinfektionsmittel oder -komponenten. Der Begriff Mittel bezeichnet in diesem Kontext anwendungsfertige Mittel, die als Einkomponentensystem zur Reinigung und/oder Desinfektion eingesetzt werden. Der Begriff Komponenten bedeutet in diesem Zusammenhang, dass für einen Reinigungs- und/oder Desinfektionsvorgang zwei oder mehr Komponenten aus zwei oder mehr Vorratsbehältern zu einem Reinigungs- und/oder Desinfektionsmittel angemischt werden. Die Vorratsbehälter können die jeweiligen Mittel und/oder Komponenten in anwendungsfertiger Konzentration enthalten, d.h. diese Mittel bzw. Komponenten können ohne Vorverdünnung in die Waschflotte der jeweiligen Aufbereitungsvorrichtung eindosiert werden.

Jeder Aufbereitungsvorrichtung ist wenigstens ein Vorlagebehälter und wenigstens eine Dosierpumpe zur Dosierung von Flüssigkeit in diesen Vorlagebehälter zugeordnet. In dem Vorlagebehälter werden Reinigungs- und/oder Desinfektionsmittel bzw. -komponenten für die unmittelbare Verwendung vorgehalten. Sofern eine Aufbereitungsvorrichtung zwei oder mehr flüssige Komponenten für die Reinigung/Desinfektion einsetzt, sind vorzugsweise dementsprechend zwei oder mehr Vorlagebehälter zugeordnet.

Da die erfindungsgemäße Aufbereitungseinheit bevorzugt eine Mehrzahl (wenigstens zwei) Vorratsbehältern und zugehörigen Vorlagebehälter aufweist, können miteinander bei der Lagerung nicht kompatible Komponenten eines Reinigungsmittels (beispielsweise alkalische Komponente einerseits und Enzyme andererseits) dennoch in Form von Konzentraten in separaten Vorratsbehältern bevorratet werden und in den zugehörigen Vorlagebehältern in das Reinigungs- und Desinfektionsgerät dosiert werden. Durch die Verdünnung beim Eindosieren in einen Tank des Reinigungs- und Desinfektionsgeräts stellen sich Konzentrationen ein, in denen bei hochkonzentrierter Lagerung nicht miteinander verträgliche Komponenten insoweit kompatibel sind, dass sie im Waschtank über die vorgesehene Dauer des Reinigungs-/Desinfektionsvorgangs die vorgesehene Wirkung entfalten.

In jeder Zufuhrleitung zu dem jeweiligen Vorlagebehälter ist eine Dosierpumpe angeordnet. Diese ist bevorzugt so ausgebildet, dass sie auch geringe Mengen Konzentrat mit hinreichender Genauigkeit in den Vorlagebehälter eindosieren kann. Die im Vorlagebehälter enthaltenen Mittel werden mittels Zufuhrleitungen zu den Reinigungs- und Desinfektionsgeräten geführt. In jeder dieser Zufuhrleitungen ist wiederum eine Dosierpumpe angeordnet; bevorzugt handelt es sich um eine Dosierpumpe des jeweiligen Reinigungs- und Desinfektionsgerätes.

Erfindungsgemäß ist vorgesehen, dass zwischen Vorratsbehälter und den wenigstens zwei Aufbereitungsvorrichtungen wenigstens ein Zwischenbehälter vorgesehen ist. Dieser Zwischenbehälter wird mit flüssigem Mittel bzw. einer flüssigen Komponente aus dem jeweiligen Vorratsbehälter gespeist. Die Ansaugleitungen der jeweiligen Aufbereitungsvorrichtungen, mittels denen die Vorlagebehälter befüllt bzw. gespeist werden, entnehmen die Flüssigkeit aus diesem Zwischenbehälter und nicht, wie im Stand der Technik üblich, direkt aus dem jeweiligen Vorratsbehälter. Der wenigstens eine Zwischenbehälter ist drucklos ausgebildet. Dies bedeutet, dass in dem Zwischenbehälter jederzeit der Umgebungsdruck/Atmosphärendruck herrscht und Füllstandsänderungen der Flüssigkeit im Zwischenbehälter darin keine Druckänderungen hervorrufen, sondern ein unmittelbarer Druckausgleich mit der Umgebung erfolgt. Diese drucklose Ausbildung des Zwischenbehälters bewirkt eine hydraulische Trennung des wenigstens einen Vorratsbehälters von den wenigstens zwei Aufbereitungsvorrichtungen. Durch diese hydraulische Trennung sind die Ansaugleitungen, mittels denen Flüssigkeit aus dem Zwischenbehälter für die Aufbereitungsvorrichtungen entnommen wird und dementsprechend die Eingänge der entsprechenden Dosierpumpen jederzeit drucklos. Dadurch kann es nicht zu Dosierungenauigkeiten bewirkt durch Druckschwankungen am Eingang der Dosierpumpen oder den typischen Dosierfehlern in einem geschlossenen hydraulischen System kommen. Solche Druckschwankungen können bei der direkten Entnahme aus einem geschlossenen Vorratsbehälter beispielsweise dann vorkommen, wenn eine gleichzeitige Dosierung in zwei oder mehr Aufbereitungsvorrichtungen stattfindet. Ein Aspekt der hydraulischen Trennung ist somit die Verbesserung des Ansaugverhaltens der Dosierpumpen der wenigstens zwei Aufbereitungsvorrichtungen.

Die hydraulische Trennung durch den Zwischenbehälter vermeidet ferner, dass es bei Betriebsstörungen und/oder Rückflüssen von Produkten aus den Ansaugleitungen der Aufbereitungsvorrichtungen zu Verunreinigungen der Produkte im Vorratsbehälter kommt. Kommt es bei der erfindungsgemäßen Aufbereitungseinheit zu solchen Rückflüssen durch die Ansaugleitungen, erfolgen diese Rückflüsse in den Zwischenbehälter und verunreinigen gegebenenfalls den Inhalt des Zwischenbehälters, durch die hydraulische Trennung ist jedoch ein Rückfluss in den eigentlichen Vorratsbehälter ausgeschlossen. Es ist in diesem Zusammenhang weiter bevorzugt, wenn die Flüssigkeiten aus dem oder den Vorratsbehältern in den Zwischenbehälter an einer Stelle eingespeist werden, die oberhalb des (höchsten) Flüssigkeitsniveaus im Zwischenbehälter liegt, so dass ein Rückstrom aus dem Zwischenbehälter in den jeweiligen Vorratsbehälter unter allen Umständen ausgeschlossen ist.

Die Erfindung hat den weiteren Vorteil, dass eine vollständige Entleerung (Restentleerung) der Vorratsbehälter möglich ist. Die Dosierpumpen, mittels denen Flüssigkeit in den Vorlagebehälter der jeweiligen Aufbereitungsvorrichtung dosiert, dürfen keine Luft ansaugen, dementsprechend müssen die zugehörigen Ansaugleitungen luftfrei bleiben. Bei einer im Stand der Technik üblichen direkten Entnahme aus einem Vorratsbehälter bedeutet dies, dass stets eine Restmenge von Flüssigkeit im Vorratsbehälter verbleiben muss, um eine Luftansaugung sicher zu vermeiden. Erfindungsgemäß ist es hingegen möglich, Vorratsbehälter vollständig zu entleeren bis zum Ansaugen von Luft, da auch dann die Flüssigkeitsentnahme aus dem Zwischenbehälter durch die Ansaugleitungen der Dosierpumpen sicherstellt, dass diese Ansaugleitungen und die entsprechenden Eingänge der Dosierpumpen jederzeit luftfrei bleiben.

Bevorzugt ist jedem Vorratsbehälter genau ein Zwischenbehälter zugeordnet. Dies bedeutet, dass in dem Zwischenbehälter keine Mischung unterschiedlicher Mittel oder Komponenten aus mehreren Vorratsbehältern erfolgt, sondern dass jeder Zwischenbehälter "sortenrein" eine Komponente oder ein Mittel enthält, wie es auch im entsprechenden Vorratsbehälter enthalten ist. Die Zuordnung genau eines Zwischenbehälters zu jedem Vorratsbehälter schließt nicht aus, dass ein Zwischenbehälter, wie unten noch näher beschrieben, aus zwei oder mehr Vorratsbehältern mit gleichem Inhalt gespeist werden kann.

Bevorzugt weist der wenigstens eine Zwischenbehälter eine Niveauüberwachung des Flüssigkeitsniveaus im Zwischenbehälter auf. Durch diese Niveauüberwachung kann sichergestellt werden, dass das Flüssigkeitsniveau im Zwischenbehälter nicht so weit fällt, dass die Ansaugleitungen der Dosierpumpen Luft ansaugen können.

Diese Niveauüberwachung ist bevorzugt zur Aktivierung einer Förderpumpe zur Förderung der flüssigen Komponente aus dem Vorratsbehälter in den wenigstens einen Zwischenbehälter ausgebildet, wenn das Flüssigkeitsniveau im Zwischenbehälter ein vorgegebenes Mindestniveau unterschreitet. Auf diese Weise ist sichergestellt, dass ein vorgegebenes Mindestniveau im Zwischenbehälter nicht unterschritten wird. Bevorzugt ist es dabei, dass die Aktivierung der Förderpumpe bis zum Erreichen eines vorgegebenen Höchstniveaus in dem wenigstens einen Zwischenbehälter erfolgt. Auf diese Art und Weise kann das Flüssigkeitsniveau im Zwischenbehälter zwischen den beiden vorgegebene Niveaus (Mindestniveau und Höchstniveau) gehalten werden.

Bevorzugt ist die erfindungsgemäße Aufbereitungseinheit so ausgebildet, dass eine Ausgabe einer Leermeldung eines Vorratsbehälters erfolgt, wenn nach Aktivierung der Förderpumpe innerhalb einer vorgegebenen Zeitspanne das vorgegebene Mindestniveau und/oder das vorgegebene Höchstniveau nicht erreicht wird. Es liegt dann ein Zustand vor, in dem die Förderpumpe zur Entnahme von Flüssigkeit aus dem Vorratsbehälter Luft ansaugt, der Vorratsbehälter mithin im Wesentlichen vollständig entleert ist. Der Zwischenbehälter und die Überwachung des Flüssigkeitsniveaus im Zwischenbehälter hat bei dieser Ausführungsform den zusätzlichen Zweck, einerseits eine vollständige Entleerung des Vorratsbehälters zu erlauben und andererseits zu signalisieren, dass diese vollständige Entleerung erfolgt ist und somit ein Austausch des Vorratsbehälters erfolgen muss.

Bevorzugt ist die Förderpumpe zur Förderung von Flüssigkeit aus dem Vorratsbehälter in den Zwischenbehälter als Schlauchpumpe ausgebildet. Eine Schlauchpumpe erlaubt eine sichere und kostengünstige Befüllung des Vorratsbehälters, sie ist zudem unempfindlich gegenüber Luftansaugung. Gemäß einer alternativen bevorzugten Ausführungsform der Erfindung kann die Förderpumpe zur Förderung von Flüssigkeit aus dem Vorratsbehälter in den Zwischenbehälter als Membranpumpe ausgebildet sein

Bevorzugt sind jedem Zwischenbehälter wenigstens zwei Vorratsbehälter zugeordnet. Diese wenigstens zwei Vorratsbehälter weisen einen identischen Inhalt auf. Dabei ist es weiter bevorzugt, dass eine Einrichtung vorgesehen ist, die nach Entleerung eines ersten Vorratsbehälters zur Umschaltung auf einen zweiten, dem gleichen Zwischenbehälter zugeordneten Vorratsbehälter ausgebildet ist. Die Zuordnung von wenigstens zwei Vorratsbehältern ermöglicht eine Behälterumschaltung im laufenden Betrieb der Aufbereitungseinheit. Wenn ein Vorratsbehälter vollständig entleert ist, kann automatisch oder manuell eine Umschaltung auf den zweiten Vorratsbehälter erfolgen. Während der Entnahme aus dem zweiten Vorratsbehälter kann der entleerte erste Vorratsbehälter gegen einen vollen Vorratsbehälter ausgetauscht werden.

Alternativ ist es erfindungsgemäß möglich, jeden Zwischenbehälter nur aus einem einzigen Vorratsbehälter zu speisen und dennoch einen Behälterwechsel ohne Betriebsunterbrechung vorzusehen, wenn der Zwischenbehälter groß genug ist, um während eines Wechsels des Vorratsbehälters die Aufbereitungsvorrichtung der Aufbereitungseinheit weiter zu speisen.

Im Rahmen der Erfindung können als Vorratsbehälter vorzugsweise Kanister (typische Füllvolumina 10 oder 20 l), Fässer (typisches Füllvolumen 200 l) oder IBC (Intermediate Bulk Container, typische Füllvolumina 600 oder 1.000 l) eingesetzt werden.

Die Füllvolumina erfindungsgemäßer Zwischenbehälter liegen vorzugsweise zwischen 0,5 und 30 l, weiter vorzugsweise 1 und 10 l.

Erfindungsgemäß ist es bevorzugt, dass der wenigstens eine Zwischenbehälter ein Füllvolumen aufweist, das 0,1 bis 50%, vorzugsweise 0,4 bis 20%, weiter vorzugsweise 0,5 bis 15% des Füllvolumens des zugeordneten Vorratsbehälters beträgt. Weitere bevorzugte Werte können beispielsweise 1%, 2%, 5% und 10% sein. Die genannten Grenzwerte von Bereichen und Einzelwerte sind beliebig kombinierbar zu neuen bevorzugten Bereichen mit entsprechenden Obergrenzen und Untergrenzen.

Die genannten bevorzugten Füllvolumina als relative Werte bezogen auf das Füllvolumen des zugeordneten Vorratsbehälters und als absolute Werte können alternativ oder kumulativ vorliegen. Das Füllvolumen sollte hinreichend groß sein, um einen sicheren Betrieb ohne Luftansaugung zu gewährleisten und gegebenenfalls auch einen Wechsel des Vorratsbehälters ohne Betriebsunterbrechung. Andererseits sollte das Füllvolumen nicht zu groß sein, damit bei einer möglichen Kontaminierung des Inhalts im Zuge einer Betriebsstörung nur eine möglichst geringe Menge Flüssigkeit verworfen werden muss.

Die Entnahme von flüssigen Komponenten aus dem wenigstens einen Vorratsbehälter erfolgt bevorzugt mittels einer Sauglanze, wie im Stand der Technik üblich. Die Sauglanze weist vorzugsweise einen weiter vorzugsweise als Schwimmerschalter ausgebildeten Niveaudetektor auf. Mittels dieses Niveaudetektors kann das Unterschreiten eines bestimmten Flüssigkeitsniveaus im Vorratsbehälter festgestellt werden.

Es ist weiter bevorzugt, dass die erfindungsgemäße Aufbereitungseinheit zur Ausgabe einer Vorleermeldung ausgebildet ist, wenn der Niveaudetektor der Sauglanze das Unterschreiten eines vorbestimmten Flüssigkeitsniveaus im Vorratsbehälter feststellt.

Diese Vorleermeldung signalisiert, dass der entsprechende Vorratsbehälter alsbald vollständig entleert sein wird, so dass geeignete Schritte bzw. Vorbereitungen zur Sicherstellung einer weiteren Flüssigkeitsversorgung getroffen werden können, beispielsweise die Bereitstellung eines neuen, gefüllten Vorratsbehälters, die Vorbereitung einer Umschaltung auf einen zweiten Vorratsbehälter mit gleichem Inhalt oder dergleichen.

Es sei angemerkt, dass eine solche Niveauunterschreitung im Vorratsbehälter bei Aufbereitungseinheiten des Standes der Technik, bei denen die Vorlagebehälter direkt aus den Vorratsbehältern gespeist werden, als (endgültige) Leermeldung gewertet wird und ein sofortiges Auswechseln des Vorratsbehälters veranlasst, um ein Ansaugen von Luft durch die Dosierpumpen zu vermeiden. Der entsprechende Behälter wird dann mit einer darin noch befindlichen Restmenge von Flüssigkeit verworfen. Diese unvollständige Entleerung kann erfindungsgemäß wie beschrieben vermieden werden.

Erfindungsgemäß können die Vorratsbehälter und die Zwischenbehälter räumlich zusammengefasst eine Dosierzentrale bilden. Die Reinigungs- und Desinfektionsgeräte können von dieser Dosierzentrale räumlich beabstandet sein, dementsprechend führen Zufuhrleitungen aus den Vorlagebehältern zu den räumlich beabstandeten Reinigungs- und Desinfektionsgeräten.

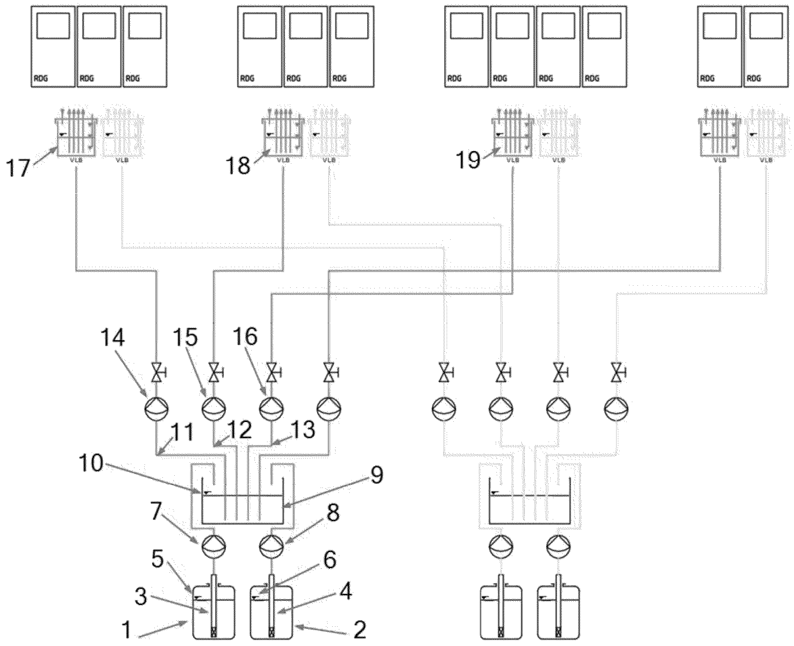

Ein Ausführungsbeispiel der Erfindung wird nachfolgend anhand der Zeichnung geschrieben. Diese zeigt schematisch wesentliche Bestandteile einer erfindungsgemäßen Aufbereitungseinheit.

In Vorratsbehältern 1, 2 befindet sich eine Flüssigkeit, die zur Reinigung und/oder Desinfektion in Aufbereitungsvorrichtung für Medizinprodukte verwendet wird. Die Entnahme der Flüssigkeit aus den Vorratsbehältern erfolgt mittels Sauglanzen 3, 4, die jeweils einen schematisch bei 5, 6 angedeuteten Schwimmerschalter als Niveaudetektor für das Flüssigkeitsniveau im Vorratsbehälter aufweisen.

Die als Schlauchpumpen ausgebildete Förderpumpen 7, 8 fördern die Flüssigkeiten in den Zwischenbehälter 9, der eine bei 10 angedeutete Niveauüberwachung aufweist. Man erkennt, dass die Flüssigkeitszufuhr in den Zwischenbehälter 9 oberhalb des Flüssigkeitsspiegels in diesem Zwischenbehälter erfolgt, um einen Rückschlag von Flüssigkeit in den entsprechenden Vorratsbehälter sicher zu vermeiden.

Aus dem Zwischenbehälter 9 wird die Flüssigkeit mittels Ansaugleitungen 11, 12, 13 und zugehörigen Dosierpumpen 14, 15, 16 in Vorlagebehälter 17, 18, 19 gefördert, die jeweils einer (in der Zeichnung nicht dargestellten) Aufbereitungsvorrichtung wie beispielsweise einem RDG zugeordnet sind. Die Vorlagebehälter weisen ebenfalls eine schematisch dargestellte (nicht mit Bezugsziffern versehene) Niveauüberwachung auf. Aus dem jeweiligen Vorlagebehälter 17, 18, 19 wird die Flüssigkeit durch nicht dargestellte Leitungen der Aufbereitungsvorrichtung zugeführt.

Die Zeichnung zeigt schematisch, wie eine Flüssigkeitskomponente aus zwei Vorratsbehältern 1, 2 drei verschiedene Aufbereitungsvorrichtungen (zugeordnet den Vorlagebehältern 17, 18, 19) zugeführt werden kann. Sofern, was die Regel ist, jeder Aufbereitungsvorrichtung zwei oder mehr Flüssigkeitskomponenten zugeführt werden, gibt es dementsprechend zwei oder mehr Einheiten aus Vorratsbehältern, Zwischenbehältern, Vorlagebehältern und den entsprechenden Pumpen, um jeweils die zweite, dritte oder weitere Flüssigkeitskomponente in die entsprechende Aufbereitungsvorrichtung zu dosieren.

Im Betrieb wird zunächst der Zwischenbehälter 9 aus dem Vorratsbehälter 1 mittels der Sauglanze 3 und der Förderpumpe 7 mit Flüssigkeit befüllt, bis die Niveauüberwachung 10 im Zwischenbehälter 9 das Erreichen eines vorgegebenen Flüssigkeitsniveaus feststellt. Im weiteren Betrieb kann mittels eines kontinuierlichen oder intermittierenden Betriebs der Förderpumpe 7 und mithilfe der Niveauüberwachung 10 das Flüssigkeitsniveau im Zwischenbehälter 9 in einem Bereich zwischen einem vorgegebenen Mindestniveau und Höchstniveau gehalten werden.

Aus dem Zwischenbehälter 9 wird mittels der Ansaugleitungen 11, 12, 13 und der entsprechenden Dosierpumpen 14, 15, 16 Flüssigkeit entnommen und in die Vorlagebehälter 17, 18, 19 eindosiert. Aus diesen Vorlagebehältern wird die Flüssigkeit anschließend in die zugehörige Aufbereitungsvorrichtung eingespeist.

Im fortlaufenden Betrieb wird der Vorratsbehälter 1 weiter entleert. Wenn mittels des Schwimmerschalters 5 das Unterschreiten eines vorbestimmten Flüssigkeitsniveaus im Vorratsbehälter 1 festgestellt wird, wird eine Vorleermeldung ausgegeben. Diese macht das Bedienungspersonal darauf aufmerksam, dass der Vorratsbehälter 1 bald vollständig entleert sein wird und das Auswechseln vorbereitet werden soll.

Es erfolgt dann eine weitere Entnahme von Flüssigkeit aus dem Vorratsbehälter 1. Zur Sicherstellung einer möglichst vollständigen Entleerung reicht die Sauglanze 3 möglichst dicht an den Boden des Behälters, bevorzugt ist es vorgesehen, dass dieser Behälter als tiefsten Punkt, an dem sich Restflüssigkeit sammelt, beispielsweise eine Bodenmulde aufweist, in die das Saugende der Sauglanze hineinreicht.

Ist der Vorratsbehälter 1 vollständig entleert, beginnt bei einer Aktivierung der Förderpumpe 7 die Sauglanze 3 Luft anzusaugen. Wenn somit im Zwischenbehälter 9 das Flüssigkeitsniveau unter ein Mindestniveau sinkt und nach Aktivierung der Förderpumpe 7 innerhalb einer vorgegebenen Zeitspanne dieses Mindestniveau und/oder ein darüber liegendes Höchstniveau nicht erreicht wird, schließt die Aufbereitungseinheit daraus, dass der Vorratsbehälter 1 vollständig entleert ist und die Förderpumpe 7 daher Luft angesaugt. Die Aufbereitungseinheit veranlasst daraufhin eine Umschaltung auf den Vorratsbehälter 2 mit der zugehörigen Sauglanze 4, dem Schwimmerschalter 6 und der Förderpumpe 8, um die fortlaufende Befüllung des Zwischenbehälters 9 damit den Betrieb der Aufbereitungsvorrichtungen der Aufbereitungseinheit sicherzustellen. Der Vorratsbehälter 1 kann dementsprechend gegen einen vollen Behälter ausgetauscht werden. Die Entnahme von Flüssigkeit aus dem Vorratsbehälter 2 erfolgt wie oben im Kontext des Vorratsbehälters 1 beschrieben.

Es sei angemerkt, dass die beschriebenen, von der Aufbereitungseinheit durchgeführten Vorgänge wie Überwachen, Pumpen, Umschalten und dergleichen mittels einer der Fachperson geläufigen und in der Zeichnung nicht dargestellten Datenverarbeitungseinheit durchgeführt werden können.

Kommt es durch Fehlfunktionen in der Aufbereitungseinheit, beispielsweise Störungen einer der Dosierpumpen 14, 15, 16 zu einem Rückfluss von Flüssigkeit aus den jeweiligen Aufbereitungsvorrichtung bzw. Vorlagebehälter 17, 18, 19, kontaminiert dieser Rückfluss den Inhalt des Zwischenbehälters 9, nicht jedoch den Inhalt der Vorratsbehälter 1, 2. Nach der Behebung einer solchen Betriebsstörung muss somit nur der Zwischenbehälter 9 neu befüllt werden, die Vorratsbehälter und deren Inhalt können weiter verwendet werden.

## Patentansprüche

1. Aufbereitungseinheit für Medizinprodukte, mit wenigstens zwei Aufbereitungsvorrichtungen für Medizinprodukte, wobei jeder Aufbereitungsvorrichtung eine Dosiervorrichtung mit wenigstens einem Vorlagebehälter (17, 18, 19) und wenigstens einer Dosierpumpe (14, 15, 16) zur Dosierung flüssiger Komponenten in den wenigstens einen Vorlagebehälter (17, 18, 19) der Aufbereitungsvorrichtung zugeordnet ist, und wobei die Aufbereitungsvorrichtungen aus Vorratsbehältern (1, 2) gespeist werden, **dadurch gekennzeichnet, dass** zwischen Vorratsbehälter (1, 2) und den wenigstens zwei Aufbereitungsvorrichtungen wenigstens ein Zwischenbehälter (9) vorgesehen ist, der mit wenigstens einer flüssigen Komponente aus wenigstens einem Vorratsbehälter (1, 2) gespeist wird und aus dem die Vorlagebehälter (17, 18, 19) der Aufbereitungsvorrichtungen gespeist werden, wobei der wenigstens eine Zwischenbehälter (9) drucklos ausgebildet ist, so dass er eine hydraulische Trennung des wenigstens einen Vorratsbehälters (1, 2) von den wenigstens zwei Aufbereitungsvorrichtungen bewirkt.

2. Aufbereitungseinheit nach Anspruch 1, **dadurch gekennzeichnet, dass** jedem Vorratsbehälter (1, 2) genau ein Zwischenbehälter (9) zugeordnet ist.

3. Aufbereitungseinheit nach Anspruch 1 oder 2, **dadurch gekennzeichnet, dass** der wenigstens eine Zwischenbehälter (9) eine Niveauüberwachung (10) des Flüssigkeitsniveaus im Zwischenbehälter aufweist.

4. Aufbereitungseinheit nach Anspruch 3, **dadurch gekennzeichnet, dass** sie zur Aktivierung einer Förderpumpe (7, 8) zur Förderung der flüssigen Komponente aus dem Vorratsbehälter (1, 2) in den wenigstens einen Zwischenbehälter (9) ausgebildet ist, wenn das Flüssigkeitsniveau im Zwischenbehälter (9) ein vorgegebenes Mindestniveau unterschreitet.

5. Aufbereitungseinheit nach Anspruch 4, **dadurch gekennzeichnet, dass** die Aktivierung der Förderpumpe (7, 8) bis zum Erreichen eines vorgegebenen Höchstniveaus in dem wenigstens einen Zwischenbehälter (9) erfolgt.

6. Aufbereitungseinheit nach Anspruch 4 oder 5, **dadurch gekennzeichnet, dass** sie zur Ausgabe einer Leermeldung eines Vorratsbehälters (1, 2) ausgebildet ist, wenn nach Aktivierung der Förderpumpe (7, 8) innerhalb einer vorgegebenen Zeitspanne das vorgegebene Mindestniveau und/oder das vorgegebene Höchstniveau nicht erreicht wird.

7. Aufbereitungseinheit nach einem der Ansprüche 1 bis 6,
**dadurch gekennzeichnet, dass** die Förderpumpe (7, 8) als Schlauchpumpe oder Membranpumpe ausgebildet ist.

8. Aufbereitungseinheit nach einem der Ansprüche 1 bis 7,
**dadurch gekennzeichnet, dass** jedem Zwischenbehälter (9) wenigstens zwei Vorratsbehälter (1, 2) zugeordnet sind.

9. Aufbereitungseinheit nach Anspruch 8, **dadurch gekennzeichnet, dass** eine Einrichtung vorgesehen ist, die nach Entleerung eines ersten Vorratsbehälters (1) zur Umschaltung auf einen zweiten dem gleichen Zwischenbehälter (9) zugeordneten Vorratsbehälter (2) ausgebildet ist.

10. Aufbereitungseinheit nach einem der Ansprüche 1 bis 9, **dadurch gekennzeichnet, dass** der wenigstens eine Zwischenbehälter (9) ein Füllvolumen aufweist, das 0,1 bis 50%, vorzugsweise 0,4 bis 20%, weiter vorzugsweise 0,5 bis 15% des Füllvolumens des zugeordneten Vorratsbehälters (1, 2) beträgt.

11. Aufbereitungseinheit nach einem der Ansprüche 1 bis 10, **dadurch gekennzeichnet, dass** der wenigstens eine Zwischenbehälter (9) ein Füllvolumen von 0,5 bis 30 l, vorzugsweise 1 bis 10 l aufweist.

12. Aufbereitungseinheit nach einem der Ansprüche 1 bis 11, **dadurch gekennzeichnet, dass** die Entnahme von flüssigen Komponenten aus dem wenigstens einen Vorratsbehälter (1, 2) mittels einer Sauglanze (3, 4) erfolgt.

13. Aufbereitungseinheit nach Anspruch 12, **dadurch gekennzeichnet, dass** die Sauglanze (3, 4) einen vorzugsweise als Schwimmerschalter ausgebildeten Niveaudetektor (5, 6) aufweist.

14. Aufbereitungseinheit nach Anspruch 13, **dadurch gekennzeichnet, dass** sie zur Ausgabe einer Vorleermeldung ausgebildet ist, wenn der Niveaudetektor (5, 6) der Sauglanze (3, 4) das Unterschreiten eines vorbestimmten Flüssigkeitsniveaus im Vorratsbehälter (1, 2) feststellt.

## Claims

1. Reprocessing unit for medical devices, comprising at least two reprocessing apparatuses for medical devices, wherein each reprocessing apparatus is assigned a dosing device having at least one holding tank (17, 18, 19) and at least one dosing pump (14, 15, 16) for dosing liquid components into the at least one holding tank (17, 18, 19) of the reprocessing apparatus, and wherein the reprocessing apparatuses are supplied from storage tanks (1, 2), **characterized in that** at least one intermediate tank (9) is provided between the storage tank (1, 2) and the at least two reprocessing apparatuses, the intermediate tank being supplied with at least one liquid component from at least one storage tank (1, 2) and the holding tanks (17, 18, 19) of the reprocessing apparatuses being supplied from the intermediate tank, wherein the at least one intermediate tank (9) is configured as an unpressurized tank, so that it provides hydraulic separation between the at least one storage tank (1, 2) and the at least two reprocessing apparatuses.

2. Reprocessing unit according to Claim 1, **characterized in that** exactly one intermediate tank (9) is assigned to each storage tank (1, 2).

3. Reprocessing unit according to Claim 1 or 2, **characterized in that** the at least one intermediate tank (9) comprises a level monitoring device (10) for monitoring the liquid level in the intermediate tank.

4. Reprocessing unit according to Claim 3, **characterized in that** it is configured to activate a delivery pump (7, 8) for conveying the liquid component from the storage tank (1, 2) into the at least one intermediate tank (9) when the liquid level in the intermediate tank (9) falls below a predetermined minimum level.

5. Reprocessing unit according to Claim 4, **characterized in that** activation of the delivery pump (7, 8) continues until a predetermined maximum level has been reached in the at least one intermediate tank (9).

6. Reprocessing unit according to Claim 4 or 5, **characterized in that** it is configured so that an empty-container message for a storage tank (1, 2) is generated if, after activation of the delivery pump (7, 8), the predetermined minimum level and/or the predetermined maximum level is not reached within a predetermined period of time.

7. Reprocessing unit according to any one of Claims 1 to 6, **characterized in that** the delivery pump (7, 8) is configured as a peristaltic pump or a diaphragm pump.

8. Reprocessing unit according to any one of Claims 1 to 7, **characterized in that** at least two storage tanks (1, 2) are assigned to each intermediate tank (9).

9. Reprocessing unit according to Claim 8, **characterized in that** a device is provided which, after a first storage tank (1) has been emptied, is configured to switch over to a second storage tank (2) assigned to the same intermediate tank (9).

10. Reprocessing unit according to any one of Claims 1 to 9, **characterized in that** the at least one intermediate tank (9) has a fill volume corresponding to 0.1 to 50%, preferably 0.4 to 20%, more preferably 0.5 to 15%, of the fill volume of the associated storage tank (1, 2).

11. Reprocessing unit according to any one of Claims 1 to 10, **characterized in that** the at least one intermediate tank (9) has a fill volume of 0.5 to 30 L, preferably 1 to 10 L.

12. Reprocessing unit according to any one of Claims 1 to 11, **characterized in that** liquid components are withdrawn from the at least one storage tank (1, 2) by means of a suction lance (3, 4).

13. Reprocessing unit according to Claim 12, **characterized in that** the suction lance (3, 4) comprises a level detector (5, 6), preferably configured as a float switch.

14. Reprocessing unit according to Claim 13, **characterized in that** it is configured to generate a low-level warning when the level detector (5, 6) of the suction lance (3, 4) detects that the liquid level in the storage tank (1, 2) has fallen below a predetermined level.

## Revendications

1. Unité de conditionnement pour produits médicaux, comprenant au moins deux dispositifs de conditionnement pour produits médicaux, un dispositif de dosage comprenant au moins un réservoir (17, 18, 19) et au moins une pompe de dosage (14, 15, 16) destinée au dosage de composants liquides dans l'au moins un réservoir (17, 18, 19) du dispositif de conditionnement étant associé à chaque dispositif de conditionnement, et les dispositifs de conditionnement étant alimentés à partir de réservoirs (1, 2), **caractérisée en ce qu'**au moins un récipient intermédiaire (9) est prévu entre le réservoir (1, 2) et les au moins deux dispositifs de conditionnement, lequel est alimenté en au moins un composant liquide provenant d'au moins un réservoir (1, 2) et à partir duquel sont alimentés les réservoirs (17, 18, 19) des dispositifs de conditionnement, l'au moins un récipient intermédiaire (9) étant configuré sans pression, de sorte qu'il produit une séparation hydraulique de l'au moins un réservoir (1, 2) des au moins deux dispositifs de conditionnement.

2. Unité de conditionnement selon la revendication 1, **caractérisée en ce qu'**exactement un récipient intermédiaire (9) est associé à chaque réservoir (1, 2).

3. Unité de conditionnement selon la revendication 1 ou 2, **caractérisée en ce que** l'au moins un récipient intermédiaire (9) possède une surveillance de niveau (10) du niveau de liquide dans le récipient intermédiaire.

4. Unité de conditionnement selon la revendication 3, **caractérisée en ce qu'**elle est configurée pour activer une pompe d'alimentation (7, 8) destinée à refouler le composant liquide hors du réservoir (1, 2) dans l'au moins un réservoir intermédiaire (9) lorsque le niveau de liquide dans le réservoir intermédiaire (9) devient inférieur à un niveau minimal prédéfini.

5. Unité de conditionnement selon la revendication 4, **caractérisée en ce que** l'activation de la pompe d'alimentation (7, 8) a lieu jusqu'à ce qu'un niveau maximal prédéfini soit atteint dans l'au moins un récipient intermédiaire (9).

6. Unité de conditionnement selon la revendication 4 ou 5, **caractérisée en ce qu'**elle est configurée pour délivrer une notification de vide d'un réservoir (1, 2) si, après l'activation de la pompe d'alimentation (7, 8), le niveau minimal prédéfini et/ou le niveau maximal prédéfini n'est pas atteint dans un intervalle de temps prédéfini.

7. Unité de conditionnement selon l'une des revendications 1 à 6, **caractérisée en ce que** la pompe d'alimentation (7, 8) est réalisée sous la forme d'une pompe à tuyau ou d'une pompe à membrane.

8. Unité de conditionnement selon l'une des revendications 1 à 7, **caractérisée en ce qu'**au moins deux réservoirs (1, 2) sont associés à chaque récipient intermédiaire (9).

9. Unité de conditionnement selon la revendication 8, **caractérisée en ce qu'**un appareil est prévu, lequel est configuré pour, après qu'un premier réservoir (1) ait été vidé, permuter sur un deuxième réservoir (2) associé au même récipient intermédiaire (9).

10. Unité de conditionnement selon l'une des revendications 1 à 9, **caractérisée en ce que** l'au moins un récipient intermédiaire (9) présente un volume de remplissage qui est de 0,1 à 50 %, de préférence de 0,4 à 20 %, de manière particulièrement préférée de 0,5 à 15 % du volume de remplissage du réservoir (1, 2) associé.

11. Unité de conditionnement selon l'une des revendications 1 à 10, **caractérisée en ce que** l'au moins un récipient intermédiaire (9) présente un volume de remplissage de 0,5 à 30 l, de préférence de 1 à 10 l.

12. Unité de conditionnement selon l'une des revendications 1 à 11, **caractérisée en ce que** le prélèvement de composants liquides hors de l'au moins un réservoir (1, 2) s'effectue au moyen d'une canne d'aspiration (3, 4).

13. Unité de conditionnement selon la revendication 12, **caractérisée en ce que** la canne d'aspiration (3, 4) possède un détecteur de niveau (5, 6), de préférence réalisé sous la forme d'un interrupteur à flotteur.

14. Unité de conditionnement selon la revendication 13, **caractérisée en ce qu'**elle est configurée pour délivrer une pré-notification de vide lorsque le détecteur de niveau (5, 6) de la canne d'aspiration (3, 4) constate le franchissement vers le bas d'un niveau de liquide prédéterminé dans le réservoir (1, 2).
